# FASCICULE DE BREVET EUROPEEN

(11) **EP 3 224 232 B1**
(45) Date de publication et mention de la délivrance du brevet: **26.09.2018**
(21) Numéro de dépôt: 15808746.0
(22) Date de dépôt: 26.11.2015
(51) Int. Cl.: C07C 51/43, C07C 55/10, C12P 7/46, C07C 51/02, C07C 51/47

(54) **PROCEDE DE RECUPERATION DE CRISTAUX D'ACIDE SUCCINIQUE AVEC MISE EN OEUVRE DE TENSIOACTIFS AU COURS DE LA CRISTALLISATION ET CRISTAUX OBTENUS**
VERFAHREN ZUR RÜCKGEWINNUNG VON BERNSTEINSÄUREKRISTALLEN UNTER VERWENDUNG VON TENSIDEN WÄHREND DER KRISTALLISATION UND DARAUS ERHALTENE KRISTALLE
METHOD FOR RECOVERING SUCCINIC ACID CRYSTALS USING SURFACTANTS DURING CRYSTALLISATION, AND RESULTING CRYSTALS

(30) Priorité: 26.11.2014 FR 1461470
(43) Date de publication de la demande: 04.10.2017
(73) Titulaire: Roquette Frères, 62136 Lestrem (FR)
(72) Inventeur: DUFLOT, Pierrick, 62136 La Couture (FR); LANOS, Pierre, 59480 La Bassee (FR); BOIT, Baptiste, 59253 La Gorgue (FR); DEHAY, Frédérick, 62840 Laventie (FR); ROSSI, Laurent, 62000 Arras (FR)
(74) Mandataire: Cabinet Plasseraud
(86) Numéro de dépôt international: PCT/FR2015/053226
(87) Numéro de publication internationale: WO 2016/083749

(56) Documents cités:
- WO-A1-01/07389
- WO-A1-2011/064151

## Description

### Domaine de l'invention

La présente invention a pour objet un procédé amélioré de récupération de cristaux d'acide succinique, dont une des originalités repose sur une double cristallisation et la mise en oeuvre de tensioactifs au cours de la première étape de cristallisation. De manière très avantageuse, on obtient au final des cristaux avec un indice de coloration b dans le référentiel (L, a, b) inférieur ou égal à 1,00 ce qui est le gage de produits d'une excellente stabilité, susceptible d'être utilisés pour fabriquer des matériaux polymères sans altérer leur coloration.

### Etat de l'art

L'acide succinique (ou acide butanedioïque) est un acide organique avec deux groupes carboxyles, de formule semi-développée COOH-CH₂-CH₂-COOH, qui trouve aujourd'hui de nombreuses applications dans les domaines cosmétiques, agroalimentaires, pharmaceutiques, textiles et dans l'industrie des matériaux plastiques. A titre d'exemple pour cette dernière application, il peut être utilisé comme intermédiaire de synthèse dans la fabrication de 1,4 butanediol, de tétrahydrofurane et de gamma-butyrolactone.

Initaielement, l'acide succinique était produit à synthétisé grâce à des procédés centrés sur des matières premières d'origine fossile. Des alternatives à ces méthodes ont ensuite été développées, faisant intervenir des produits bio-sourcés. A ce titre, l'acide succinique peut aujourd'hui être produit à partir de matières premières renouvelables : en l'occurrence par le biais de processus de fermentation.

Divers microorganismes sont connus pour leur capacité à produire de l'acide succinique selon cette voie comme *Actinobacillus succinogenes, Mannheimia succiniciproducens, Escherichia coli,* ou *Aspergillus niger* et *Saccharomyces cerevisiae.* Ceci étant, les produits de fermentation contiennent des quantités substantielles d'impuretés (débris de biomasse, sucres, acides aminés, oligoéléments, sels...) qui sont autant de précurseurs de coloration susceptibles par leur présence, même à l'état de traces dans le produit final, d'influer sur la qualité de l'acide succinique purifié, et par voie de conséquence, sur la qualité du polymère synthétisé à partir de l'acide succinique purifié.

L'homme du métier connaît aujourd'hui de nombreux procédés de fabrication d'acide succinique qui mettent en oeuvre diverses étapes de purification et / ou de décoloration.

On peut citer les procédés mettant en oeuvre des solvants. C'est par exemple le cas de la méthode décrite dans le document US 6,265,190 qui enseigne la récupération d'acide succinique par addition de sulfate d'ammonium dans un milieu de fermentation concentré en ions succinate. Du méthanol est ensuite utilisé pour purifier l'acide succinique obtenu.

La nano filtration est également une technique reconnue pour purifier les cristaux d'acide succinique : le document CN 101475464 en est un exemple.

On connaît aussi les procédés utilisant des résines échangeuses d'ions ou du charbon actif. Ainsi, le document US 5,168,055 enseigne la réaction entre l'acide sulfurique et un milieu de fermentation riche en succinate de calcium, de manière à produire conjointement du sulfate de calcium et de l'acide succinique. Ce dernier est purifié grâce à une résine cationique forte et une résine anionique faible. Le document WO 2013 / 169447 décrit la mise en oeuvre de résines non fonctionnalisées. Le document WO 2009 / 082050 propose de traiter le milieu de fermentation avant cristallisation, au moyen de charbon actif.

Deux demandes de brevet déposées par la société Demanderesse sont également une illustration des méthodes citées dans le paragraphe précédent : les demandes de brevets WO 2011 / 064151 et WO 2013 / 144471. Il convient de noter que les procédés ici décrits associent l'utilisation de charbon actif avec la mise en oeuvre de résines, tout en s'appuyant sur une double cristallisation.

Il existe aussi un pan de l'art antérieur qui combine la nano filtration et des techniques basées sur des résines échangeuses d'ions et / ou du charbon actif. Dans cet ensemble, on peut citer les documents US 2012 / 0289742, CN 101215583, US 2010 / 0317891 et WO 2014 / 106532.

Ceci étant, aux meilleurs des connaissances de la société Demanderesse, aucune des méthodes existantes à la production d'acide succinique n'est susceptible de conduire à un produit présentant un niveau d'impuretés colorantes suffisamment faible, de manière à obtenir au final des polymères dont la couleur n'est pas altérée.

Plus précisément, la société Demanderesse a déjà montré dans la demande de brevet WO 2013 / 144471 qu'une mesure colorimétrique appropriée reflétait parfaitement bien le niveau d'impuretés responsables d'une décoloration de l'acide succinique, cette décoloration étant parfaitement reliée à une décoloration du polymère final fabriqué avec cet acide succinique. On rappelle que toute mesure colorimétrique est basée sur la théorie des couleurs opposées qui spécifie que les réponses des cônes (cellules de la rétine de l'oeil humain responsables de la vision des couleurs) aux couleurs rouge, verte et bleu sont recombinées en signaux opposés « noir-blanc », « rouge-vert » et « jaune-bleu » lorsque transmis au cerveau par le nerf optique. Cette mesure repose notamment sur les échelles de couleur largement utilisées dans les industries alimentaires et les industries des polymères, appelées échelles « L », « a », « b » de HUNTER. On parle également de « référentiel (L, a, b) ».

Dans la demande de brevet précitée, il a été notamment démontré qu'on parvenait au mieux à un indice « b » de 1,1 (mesuré dans le référentiel L, a, b) pour des cristaux d'acide succinique, qui eux-même permettaient de fabriquer un polymère de type PBS (poly butylène succinate) ayant une « qualité colorimétrique acceptable », cette dernière étant mesurée à travers l'indice de jaune « YI » (selon la norme ASTM D1925).

Or, à la suite de nombreux travaux, la société Demanderesse est parvenue à mettre au point un procédé particulièrement simple, conduisant à des cristaux d'acide succinique présentant un indice colorimétrique « b », tel que mesuré dans le référentiel (L, a, b), encore jamais atteint à savoir inférieur ou égal à 1,00 et même dans certains cas inférieur ou égale à 0,90 et très préférentiellement inférieur ou égal à 0,80.

Ce procédé repose notamment sur l'introduction de tensioactifs au cours d'une première étape de cristallisation. Celle-ci est suivie par des étapes ultérieures de dissolution des cristaux formés, de purification de la solution obtenue notamment par traitement avec du charbon actif et / ou des résines échangeuses d'ions, par une seconde étape de cristallisation, puis par le séchage et le refroidissement des cristaux obtenus.

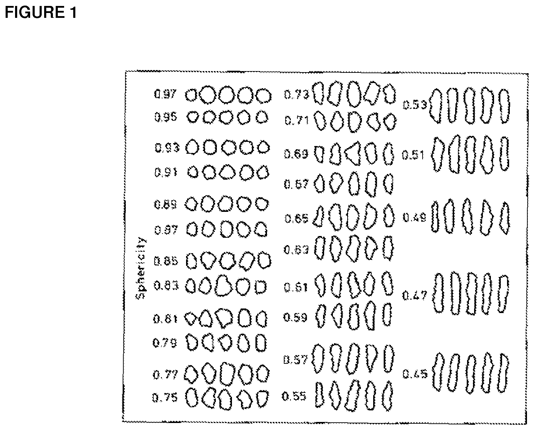

Par ailleurs, le procédé selon la présente invention met en oeuvre 2 étapes de cristallisation. De manière avantageuse, la première étape de cristallisation conduit à des cristaux sous forme de « billes », dont on verra qu'elles améliorent certains aspects du procédé en question. On oppose notamment les « billes » aux « aiguilles » qui sont l'autre forme la plus répandue selon laquelle on peut obtenir des cristaux d'acide succinique. Néanmoins, lesdites billes peuvent être définies de manière positive à travers un indice dit « de sphéricité ». Cet indice est dans toute la présente Demande déterminé de manière visuelle à partir de la définition standard de forme de Rittenhouse (« Agglomération in industry », W. Pietsch, p.600 vol. 2, 2005 Wiley-VCH), qui consiste à associer un indice de sphéricité à une appréciation visuelle de la forme des particules observées. La figure 1 fait notamment apparaître la grille d'évaluation et d'attribution des notes selon ce test.

Dans le cas de la présente invention, la mise en oeuvre de tensio-actif conduit à la formation de cristaux sous forme de billes au niveau de la première étape de cristallisation, cette forme étant conservée par la suite dans le procédé. On entend ici par billes de cristaux présentant un indice de sphéricité, tel que mesuré selon le test de Rittenhouse, au moins égal à 0,70, préférentiellement au moins égal à 0,75, très préférentiellement au moins égal à 0,85.

Or, il est particulièrement avantageux de disposer de cristaux sous forme de billes, et non sous forme d'aiguilles, dans la mesure où il est plus facile de séparer les premiers des eaux mères de cristallisation lors d'une étape de centrifugation ou de filtration. Ceci est notamment rapporté dans le document « Chirality in Industry II: Developments in the Commercial Manufacture and Applications of Optically Active Compounds » (A. N. Collins, G. N. Sheldrake, J. Crosby, John Wiley & Sons, 1997 - p 125).

Il est également connu que les cristaux sous forme d'aiguilles sont plus difficiles à rincer après cristallisation et la séparation entre lesdits cristaux et le flux d'eaux mères : cette étape de rinçage consiste à éliminer les eaux mères résiduelles à la surface des cristaux. On pourra se reporter à cet égard aux documents « Handbook of Industrial Drying » (Arun S. Mujumdar, 4th Edition, CRC Press, p 1273, 64.1.5 Crystal purity) et Crystal Shape Enhancement : a Processing Solution to a Product Problem (Snyder, R. C., Studenar, S., Doherty, M. F., AlChE 2006 Annual Meeting).

De plus, les cristaux sous forme d'aiguilles ont tendance à présenter plus d'impuretés sous formes d'eaux mères incluses, c'est-à-dire d'eaux mères qui se trouvent physiquement emprisonnées dans le cristal. Il en résulte donc un moins bon pouvoir de purification que le tensioactif permet d'améliorer (« Handbook of Industrial Crystallization, 2nd Edition, Allan S.Myerson, p 259).

Au regard de l'état de la technique, un tel résultat est particulièrement surprenant. En effet, on connaît le document WO 01 / 07389 qui enseigne l'utilisation de tensioactifs au moment de la formation de cristaux d'acide succinique. Il est clairement expliqué dans ce document que la mise en oeuvre de tensioactifs conduit alors à la formation de cristaux sous forme d'aiguilles, et non pas de billes.

La société demanderesse a également montré que l'ajout de tensioactif permet le recyclage d'une partie des eaux mères de cristallisation et de lavage en tête de première cristallisation ce qui améliore le rendement de récupération d'acide succinique et permet d'obtenir un « b » conforme à l'invention. Le rendement de récupération est défini comme le rapport de la masse de cristaux d'acide succinique obtenue après séchage sur la masse d'acide succinique contenue dans le jus de fermentation acidifié (avant l'étape b). Ce rendement peut aussi être exprimé en pourcentage.

Aussi, non seulement la société demanderesse a-t-elle dû sélectionner le procédé de départ ad-hoc parmi toutes les méthodes jusqu'alors accessibles : celui décrit dans les documents WO 2011 / 064151 et WO 2013 / 144471, à base d'une double cristallisation. Ensuite, elle est allée à l'encontre de ce qu'enseignait l'état de la technique : en mettant en oeuvre des tensioactifs dans une étape de cristallisation de l'acide succinique pour en améliorer la qualité en diminuant très largement le nombre d'impuretés colorantes, mais aussi afin de privilégier la formation de cristaux sous forme de billes et non d'aiguilles.

Elle a aussi montré que les tensioactifs devaient être employés au cours de la première étape de cristallisation et non pendant la seconde. L'utilisation de tensioactifs au cours de la seconde étape de cristallisation génère en effet à nouveau des aiguilles et des valeurs de « b » non satisfaisantes. Enfin, elle a démontré que les produits obtenus présentaient un indice « b » (dans le référentiel L, a, b) inférieur à ceux obtenus à travers les procédés de l'art antérieur ne faisant intervenir qu'une seule cristallisation et la combinaison de différentes étapes à base de résines échangeuses d'ions et de charbon actif. Elle a montré que cet indice était inférieur ou égal à 1,00 avantageusement inférieur ou égal à 0,90 et très préférentiellement inférieur ou égal à 0,80.

### Résumé de l'invention

Aussi, un premier objet de la présente invention consiste en un procédé de fabrication de cristaux d'acide succinique à partir d'un milieu de fermentation contenant de l'acide succinique, comprenant les étapes de :
a) porter le milieu de fermentation à un pH compris entre 1,0 et 4,0,
b) cristalliser l'acide succinique du milieu de fermentation issu de l'étape a) de manière à former des cristaux d'acide succinique, séparer les cristaux d'acide succinique des eaux mères de cristallisation puis laver les cristaux obtenus avec de l'eau,
c) dissoudre les cristaux d'acide succinique obtenus suite à l'étape b) dans de l'eau à une température comprise entre 30 °C et 70 °C de manière à obtenir une solution contenant de l'acide succinique dissout,
d) purifier la solution d'acide succinique obtenue à l'étape c) par un traitement sur charbon actif et sur résine échangeuses d'ions,
e) cristalliser l'acide succinique contenu dans la solution obtenue à l'étape d) de manière à former des cristaux d'acide succinique , séparer ensuite les cristaux d'acide succinique des eaux mères de cristallisation puis laver les cristaux obtenus avec de l'eau,
f) sécher les cristaux d'acide succinique à une humidité inférieure à 0,5 % et les refroidir à une température inférieure à 30°C,
caractérisé en ce qu'on introduit avant et / ou pendant l'étape b) au moins un tensioactif. De manière préférée, ledit tensioactif est introduit pendant l'étape b).

Dans toute la présente demande, on entend par tensioactif un composé qui modifie la tension superficielle entre deux surfaces.

Ledit tensioactif est préférentiellement choisi parmi les tensioactifs non ioniques, et préférentiellement parmi les polysorbates ayant un HLB supérieur à 15, par exemple de type Tween 20, parmi les tensioactifs à base de copolymère bloc d'oxyde d'alkylènes, par exemple de type Erol 18 (OUVRE PMC) ou Supra NS 1342 (HYPRO-Food), préférentiellement copolymères bloc d'oxyde de propylène et d'oxyde d'éthylène et parmi ceux présentant notamment des propriétés antimoussantes, sans pour autant que cette liste soit exhaustive.

La première étape a) du procédé selon l'invention consiste donc à porter le milieu de fermentation à un pH compris entre 1,0 et 4,0. Le pH peut notamment être porté à une valeur comprise entre 1,5 et 3,5 et préférentiellement entre 1,5 et 3,0.

Le milieu de fermentation contient typiquement des bactéries choisies parmi les souches bactériennes du genre *Mannheimia, Anaerobiospirillum, Bacillus,* ou *Escherichia,* ou a parmi les cellules fongiques. Les souches fongiques peuvent être choisies parmi *Saccharomyces cervisiae, Saccharomyces uvarum, Saccharomyces bayanus, Schizosaccharomyces pombe, Aspergillus niger, Penicillium chrysogenum, P. symplissicum, Pichia stipidis, Kluyveromyces marxianus, K. lactis, K. thermotolerans, Yarrowia lipolytica, Candida sonorensis, C. glabrata, Hansenula polymorpha, Torulaspora delbrueckii, Brettanomyces bruxellensis, Rhizopus orizae, Issatchenkia orientalis* ou *Zygosaccharomyces bailii.* Les souches bactériennes peuvent être choisies parmi *Mannheimia succiniciproducens, Anaerobiospirillum succiniciproducens Bacillus amylophylus, B. ruminucola* ou *col.*

Le milieu de fermentation consiste en tout milieu de fermentation susceptible de générer de l'acide succinique. Il peut notamment contenir une source de carbone comme le glucose, fructose, galactose, xylose, arabinose, sucrose, lactose, raffinose ou le glycerol.

La fermentation peut être de nature aérobique ou anérobique, ou dans des conditions particulières de carence en oxygène, ou résulte d'une combinaison de ces conditions, comme décrit dans le document WO 2009 / 083756.

Usuellement, on introduit ensuite un agent de neutralisation dans le milieu de fermentation, comme par exemple l'hydroxyde de potassium ou de sodium.

La régulation du pH dans la zone désirée, c'est-à-dire entre 1,0 et 4,0, préférentiellement entre 1,5 et 3,5, très préférentiellement entre 2,0 et 3,0, peut être effectuée par tous les moyens accessibles à l'homme du métier et susceptibles de faire évoluer le pH dans ce domaine. On pourra citer l'électrodialyse dipolaire en combinaison avec des résines cationiques fortes ou faibles (respectivement par exemple des résines du type polystyrene divinylbenzene (DVB) avec des groupements sulfoniques ou des résines à base des acides maléiques et fumariques), les résines cationiques faibles ou fortes utilisées seules, ou l'acidification par ajout direct d'acide chlorhydrique ou d'acide sulfurique.

A l'issue de l'étape a), le jus de fermentation acidifié présente typiquement une matière sèche comprise entre 5 % et 10 % en poids. Ce jus est alors concentré par évaporation, à une matière sèche comprise entre 15 % et 50 %, préférentiellement entre 20 % et 40 % en poids, très préférentiellement entre 30 % et 35 % en poids.

La deuxième étape b) du procédé selon l'invention consiste à cristalliser l'acide succinique du milieu de fermentation issu de l'étape a) de manière à former des cristaux d'acide succinique, puis à séparer les cristaux d'acide succinique des eaux mères de cristallisation et enfin à laver les cristaux d'acide avec de l'eau. La cristallisation intervient selon toutes les méthodes bien connues de l'homme du métier, soit en batch soit en continu et par refroidissement, notamment par contact direct avec le milieu de cristallisation ou par flash cooling.

La séparation entre les cristaux et les eaux mères peut être réalisée par toutes les techniques bien connues de l'homme du métier, et notamment par filtration ou centrifugation.

Le lavage quant à lui, est réalisé à l'eau, préférentiellement avec de l'eau déminéralisée, à une température comprise entre 15 °C et 25 °C, préférentiellement à environ 20 °C.

En outre, il est avantageux de recycler une partie des eaux mères de cristallisation et de lavage en tête de l'étape b). Ce recyclage porte sur au plus 70 % en poids de la totalité des eaux mères et de rinçage, plus préférentiellement sur 20 % à 60 % plus préférentiellement sur 30 % à 50 % en poids de celles-ci.

Cette étape est notamment caractérisée en ce qu'au moins un tensioactif est introduit pendant cette étape et / ou avant cette étape, c'est-à-dire directement dans le milieu de fermentation avant cristallisation. Le ou les tensioactifs sont introduits en batch ou en continu par pompe l'intermédiaire d'une pompe doseuse. La quantité de tensioactif est comprise préférentiellement entre 100 ppm à 5000 ppm, de préférence entre 500 ppm et 3000 ppm, plus préférentiellement entre 1000 ppm et 2000 ppm, par rapport à la masse de solution d'acide succinique provenant de l'étape a) et arrivant en tête de l'étape b).
Dès cette étape b), il faut noter que les cristaux d'acide succinique obtenus présentent généralement un indice de sphéricité, tel que mesuré selon le test de Rittenhouse, au moins égal à 0,70, préférentiellement au moins égal à 0,75, et très préférentiellement au moins égal à 0,85.

La troisième étape c) du procédé selon l'invention consiste à dissoudre les cristaux d'acide succinique obtenus suite à l'étape b) dans de l'eau, de préférence de l'eau déminéralisée, à une température comprise entre 30 °C et 70 °C de manière à obtenir une solution contenant de l'acide succinique dissout. L'acide succinique est dissout de manière à obtenir une matière sèche comprise entre 5 % et 50 % en poids du poids total de ladite solution, préférentiellement entre 10 % et 20 %.

La quatrième étape d) du procédé selon l'invention consiste à purifier la solution d'acide succinique obtenue à l'étape c) par un traitement sur charbon actif et sur résine échangeuses d'ions. Le charbon en question peut être sous forme de poudre, ou granulaire, plus préférentiellement sous forme granulaire sur une colonne fonctionnant en lit fixe ou en lit mouvant. Les résines échangeuses d'ions (IEX) peuvent être des résines cationiques fortes comme les résines polystyrene divinylbenzene (DVB) de type cationique forte avec des groupements sulfoniques et de type anioniques faibles avec des groupements amines quaternaires ou tertiaires.
La cinquième étape e) consiste à cristalliser l'acide succinique contenu dans la solution obtenue à l'étape d) pour récupérer de l'acide succinique sous forme de cristaux et à séparer des cristaux d'acide succinique et des eaux mères de cristallisation et laver les cristaux d'acide succinique obtenu avec de l'eau.
Les étapes de cristallisation, de séparation et de lavage peuvent avoir lieu selon les mêmes préconisations que pour la première étape de cristallisation. En outre, il est avantageux de recycler la totalité des eaux mères de cristallisation et de lavage de l'étape e) en tête de l'étape b).
La sixième étape f) consiste à sécher les cristaux d'acide succinique à une humidité inférieure à 0,5 %, préférentiellement inférieure à 0,4 %, très préférentiellement inférieure à 0,3 % en poids d'eau par rapport au poids sec d'acide succinique, et à les refroidir à une température inférieure à 30°C, préférentiellement inférieure à 25 °C.
Le procédé selon l'invention permet d'obtenir les cristaux d'acide succinique décrits ci-après. Ainsi, un autre objet de la présente invention consiste en des cristaux d'acide succinique obtenu à partir d'un milieu de fermentation contenant de l'acide succinique, caractérisés en ce qu'ils présentent un indice de coloration b, mesuré dans le référentiel L, a, b, inférieur ou égal à 1,00 préférentiellement inférieur ou égal à 0,90, très préférentiellement inférieur ou égal à 0,80 et en ce qu'ils présentent un indice de sphéricité, tel que mesuré selon le test de Rittenhouse, au moins égal à 0,70, préférentiellement au moins égal à 0,75, et très préférentiellement au moins égal à 0,85.
Cette composition peut notamment être une poudre constituée de cristaux d'acide succinique ayant la valeur d'indice « b » précitée.
Dans toute la présente Demande, la mesure du paramètre « b » est effectuée comme suit :
1) on prépare une poudre cristalline d'acide succinique présentant moins de 1 % de teneur en eau résiduelle, notamment en séchant la poudre de manière à obtenir cette teneur ;
2) on place un échantillon de ladite poudre cristalline dans une étuve à 220 °C pendant 2 h,
3) on broie et on tamise la poudre cristalline ainsi traitée, de manière à ce que sa répartition granulométrique soit la suivante, telle que déterminée sur tamis vibrant RETSCH :
   - de 0 à 10 %, de préférence de 4 à 6 % en poids des particules ayant une taille supérieure à 500 µm,
   - de 20 % à 40 %, de préférence de 25 % à 35 % en poids des particules ayant une taille comprise entre 200 µm et 500 µm
   - de 50 % à 75 %, de préférence comprise de 55 % à 70 % en poids des particules ayant une taille inférieure à 200 µm,
4) on réalise la mesure de la couleur dans un spectrocolorimètre de la poudre broyée et tamisée et déterminer la valeur moyenne de l'indice « b »

La mesure est réalisée 10 fois sur le même échantillon ce qui donne une incertitude de +/-0,05 sur le résultat. Ladite mesure est réalisée sur un spectrocolorimètre permettant la mesure de la réflexion de la longueur d'onde entre 400 nm et 700 nm, comme par exemple le Dataflash 100 commercialisé par la société DATACOLOR. (Ouverture de mesure : 9 mm de diamètre " ; illuminant de lecture : " C2 Deg ").

La composition selon l'invention peut comprendre au moins 50% en nombre desdits cristaux d'acide succinique, avantageusement au moins 70%, préférentiellement au moins 90%. Tout préférentiellement, la composition est essentiellement constituée des cristaux selon l'invention.
De manière avantageuse, les cristaux présentent également une teneur en sucres réducteurs inférieure à 20 ppm, préférentiellement inférieure à 10 ppm, par rapport au poids total de cristaux anhydres. Cette teneur est généralement supérieure à 0,1 ppm. Les sucres qu'on trouve sont typiquement le glucose, mannose, trehalose, l'isomaltose, le maltose, le maltulose, le gentobiose et le panose. La mesure est réalisée selon la brochure technique de 2004 de la société DIONEX « Analysis of Carbohydrates by High-PerformanceAnion-Exchange Chromatography with Pulsed Amperometric Detection (HPAE-PAD) ».
Les exemples qui suivent permettent de mieux illustrer la demande sans pour autant en limiter la portée.

### EXEMPLES

### Exemple 1

### Fermentation

On réalise un milieu de fermentation dans les conditions strictes de l'exemple 1 du document WO 2011 / 064151. Seuls diffèrent les dispositifs mis en oeuvre : l'étape de pré culture est réalisé sur un réacteur Puntbus 6 L, la phase de croissance sur un fermenteur de 7 m³ et la phase de production sur 2 fermenteurs de 70 m³. Le débit de jus de fermentation est alors d'environ 1,5 m³ / h.

La séparation de la biomasse et du jus de fermentation et réalisée par microfilitration. Cette dernière est réalisée à une température de 80 °C en batch suivi d'une étape de diafiltration. Le module est équipé de 2 carters de 25 m², céramique « Kerasep », d'une porosité égale à 0,1 µm. Le débit moyen de perméat est d'environ 2 m³/h avec une pression transmembranaire d'environ 1 bar.

### Etape a) d'acidification

Le jus de fermentation est traité sur résine cationique faible de type AMBERLITE IRC 747 à un débit de 2 BV/h à 60°C afin d'atteindre une concentration en ions divalents inférieure à 5 ppm. La régénération des résines est effectuée après avoir passé un volume de jus de fermentation compris entre 30 et 40 fois le volume de résine.

La solution obtenue est alors acidifié sur un module EDB (electrodialyse bipolaire) Aqualyzer® EDBM EUR40 commercialisé par la société d'EURODIA à un pH d'environ 3,5.

La solution est alors traitée sur une résine cationique forte de type PUROLITE C150 à un débit 2 BV/h et une température de 40°C afin d'atteindre un pH de 2,0. La régénération des résines est effectuée après avoir passé un volume de jus de fermentation compris entre 15 et 20 fois le volume de résine.

### Première cristallisation : étape b)

La solution acidifiée est concentrée sur un évaporateur sous vide à circulation forcée à plaques commercialisé par la société ALFA LAVAL à une concentration de 35 % en matière sèche et à une température de 80°C. Elle est ensuite cristallisée en continu par flash cooling sur 2 étages composés chacun d'un cristallisoir sous vide à boucle externe commercialisé par la société GEA KESTNER. Le vide dans les cristallisoirs est fixé de manière à obtenir une température sur le premier étage de 40°C et sur le deuxième étage de 20°C. Le temps de séjour est d'environ 5 h.

Dans le cas de l'utilisation du tensioactif, ce dernier est introduit en continu à l'aide d'une pompe doseuse à un débit égal à 1200 ppm par rapport au débit de solution qui alimente le cristallisoir. Le tensioactif choisi est l'EROL18 commercialisée par la société PMC OUVRIE.

La masse cristallisé est ensuite séparé sur une centrifugeuse batch de type SC 1200 commercialisée par la société ROBATEL afin de récupérer les cristaux d'acide succinique. Au cours de cette étape, les cristaux sont lavés par de l'eau déminéralisée à 20°C avec quantité égale 1 kg / kg de cristaux

Entre 0 et 70 % en poids des eaux mères de cristallisation et de lavage récupérées sur la centrifugeuse sont recyclées en tête de d'évaporateur.

### Etape c) de dissolution des cristaux

La dissolution des cristaux d'acide succinique est réalisée à une température de 45°C avec de l'eau déminéralisée de manière à obtenir une solution à 10 % de matière sèche.

### Etape d) de purification

L'étape de traitement sur charbon actif est réalisée avec un charbon granulaire de type CHEMVIRON CPG LF 12x40 sur une colonne fonctionnant en lit fixe. Le débit de solution dans la colonne est fixé à 0,5 BV / h et le volume de solution traité avant renouvellement du lit est variable en fonction des conditions opératoires et de la qualité souhaitées. Elle est comprise entre 40 et 500 fois le volume de charbon de la colonne.

L'étape de traitement sur résines échangeuses d'ions est réalisés à un débit de 2 BV / h à 60°C sur une résine cationique forte de type DOWEX 88 puis sur une résine anionique faible de type Lanxess Lewatit S4528. La régénération des résines est effectuée après avoir passé un volume de solution égale à 40 fois le volume de résine.

### Seconde cristallisation : étape e)

La solution d'acide succinique purifiée est concentrée sur un évaporateur sous vide à film tombant commercialisé par la société WIEGAND à une concentration de 30 % en matière sèche et une température de 80 °C. Elle est ensuite cristallisée en continu par flash cooling sur 2 étages composés chacun d'un cristallisoir sous vide à boucle externe commercialisé par la société GEA KESTNER. Le vide dans les cristallisoirs est fixé de manière à obtenir une température sur le premier étage de 40 °C et sur le deuxième étage de 20 °C. Le temps de séjour est d'environ 7 h.

La masse cristallisé est ensuite séparé sur une centrifugeuse batch de type SC 1200 commercialisée par la société ROBATEL afin de récupérer les cristaux d'acide succinique. Au cours de cette étape, les cristaux sont lavés par de l'eau déminéralisée à 20 °C avec une quantité de cristaux égale à 1 kg / kg.

La totalité des eaux mères de cristallisation et de lavage est récupérée sur la centrifugeuse et recyclée en tête de d'évaporateur au niveau de l'étape b)

### Etape f) de séchage

Le produit est séché sur un séchoir rotatif de manière à obtenir une humidité résiduelle égale à 0,3 % en poids d'eau par rapport au poids total de produit, puis refroidi sur lit fluidisé à une température de 25 °C

### Exemple 2

### Fermentation

On réalise un milieu de fermentation dans les conditions strictes de l'exemple 5 du document WO 2011 / 064151. Seuls diffèrent les dispositifs mis en oeuvre : l'étape de pré culture est réalisé sur un réacteur Puntbus 6 L, la phase de croissance sur un fermenteur de 7 m³ et la phase de production sur 2 fermenteurs de 70 m³.

La séparation de la biomasse et du jus de fermentation et réalisée par microfiltration. Cette dernière est réalisée à une température de 80 °C en batch suivi d'une étape de diafiltration. Le module est équipé de 2 carters de 25 m², céramique « Kerasep », d'une porosité égale à 0,1 µm. Le débit moyen de perméat est d'environ 2 m3/h avec une pression transmembranaire d'environ 1 bar.

### Etape a) d'acidification

Le jus de fermentation est alors traitée sur une résine cationique forte de type PUROLITE C150 à un débit 2 BV / h et une température de 40°C afin d'atteindre un pH de 2,0. La régénération des résines est effectuée après avoir passé un volume de jus de fermentation compris entre 15 et 20 fois le volume de résine

L'ensemble des autres étapes b) à f) est réalisé tel que décrit dans l'exemple 1.

### Exemple 3

Cet exemple correspond à la réalisation d'essais selon ou hors invention (avec ou sans tensioactif), dans un procédé à double cristallisation.

On a réalisé 6 essais, de manière à évaluer l'influence de 2 paramètres : la valeur du rapport entre le volume de solution traité et le volume de charbon dans l'étape c) (« Bed Volume » ou BV = Volume de solution / volume de charbon actif) et le % d'eaux mères de cristallisation recyclé au niveau de l'étape b).

Les essais n° 1 à 3 sont réalisés selon le protocole donné dans l'exemple 1 et sans tensioactif.
Les essais n° 4 à 6 sont réalisés selon le protocole donné dans l'exemple 2 et sans tensioactif.
Les essais n° 7 et 8 sont réalisés selon le protocole donné dans l'exemple 1, avec 1200 ppm d'EROL 18 en tant que tensioactif.
Les essais n° 9 à 11 sont réalisés selon le protocole donné dans l'exemple 2, avec 1200 ppm d'EROL 18 en tant que tensioactif.

La mesure de l'indice « b » est réalisé sur un échantillon moyen : on prélève un échantillon toutes les 12 heures et ce, pendant 15 jours, puis on mélange l'ensemble de ces prélèvements.

**Tableau 1**

| **Essais** | **BV** | **% recyclage des eaux mères %** | **Indice « b »** | **Rendement %** | **Sucres totaux (ppm)** |
|---|---|---|---|---|---|
| 1 | 50 | 0 | 1.80 | 81 | 58 |
| 2 | 100 | 0 | 2.50 | 80 | 98 |
| 3 | 50 | 50 | 4.20 | 89 | 158 |
| 4 | 50 | 0 | 1.75 | 80 | 41 |
| 5 | 100 | 0 | 2.10 | 79 | 59 |
| 6 | 50 | 50 | 3.90 | 90 | 157 |

**Tableau 2**

| **Essais** | **BV** | **% recyclage des eaux mères** | **Indice « b »** | **Rendement %** | **Sucres totaux (ppm)** |
|---|---|---|---|---|---|
| 7 | 300 | 0 | 0.71 | 82 | 4 |
| 8 | 300 | 50 | 0.91 | 90 | 16 |
| 9 | 300 | 0 | 0.65 | 81 | 2 |
| 10 | 300 | 50 | 0.87 | 90 | 10 |
| 11 | 300 | 60 | 0.95 | 94 | 16 |

La comparaison entre les tableaux 1 et 2 fait clairement apparaître l'influence positive du tensioactif sur les valeurs de l'indice « b ».
En outre, on peut jouer à la fois sur la valeur de cet indice et sur le rendement du procédé en acide succinique, à travers le recyclage de tout ou partie des eaux- mères de première cristallisation.

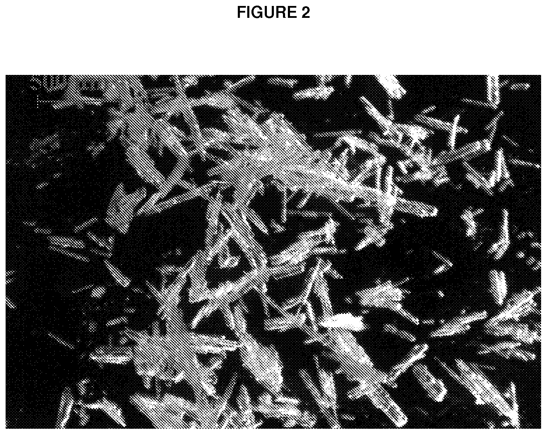

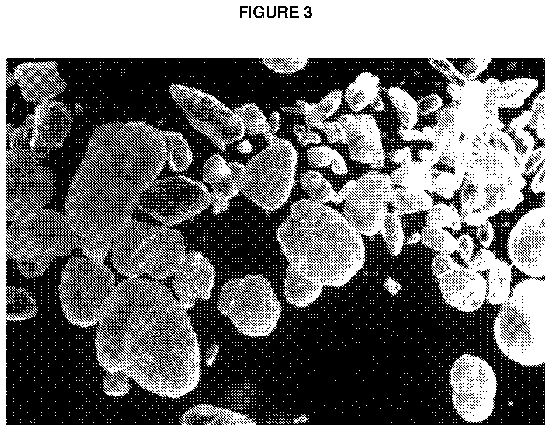

Enfin, la figure 2 fait apparaître la morphologie des cristaux obtenus sans tensioactif (essai n° 1) qui sont des aiguilles alors que la figure 3 (essai n° 7) fait apparaître des billes, présentant un indice bien supérieur à 0,70. Ces photos ont été réalisées sur un microscope LEICA EZ4HD. Ceci est particulièrement surprenant dans la mesure où il était connu du document WO 01 / 07389 que l'utilisation de tensioactifs dans un procédé de cristallisation d'acide succinique menait à la formation de cristaux d'acide succinique sous forme d'aiguilles. Or, parvenir à de tels cristaux sous forme de billes est particulièrement avantageux car, comme indiqué précédemment, la purification de l'acide succinique est alors améliorée pour différentes raisons ; par ailleurs, ces cristaux s'écoulent mieux et ont moins tendance à motter. Sans être liée par une quelconque théorie, la Demanderesse explique cette différence fondamentale de comportement lors de la cristallisation par les impuretés d'un acide succinique obtenu à partir d'un milieu de fermentation, qui sont différentes de celles de l'acide succinique pétrosourcé du document WO 01 / 07389.

### Exemple 4

Cet exemple correspond à la réalisation d'essais hors invention (avec ou sans tensioactif), dans un procédé à une seule cristallisation.

Au cours des différents essais, des cristaux sont prélevés avant l'étape c) de dissolution dans certains des essais de l'exemple précédent.
Les cristaux sont ensuite séchés sur un lit fluidisé de laboratoire RETSCH jusqu'à obtenir une humidité de 0,3 % en poids d'eau par rapport au poids total de produit.

**Tableau 3**

| **Essais** | **Indice « b »** |
|---|---|
| 1 bis | 9.01 |
| 7 bis | 5.05 |
| 4 bis | 8.27 |
| 9 bis | 4.39 |

Les résultats du tableau 3 démontrent que les procédés à une seule cristallisation, en présence de tensioactif ou non, ne conduisent pas à des valeurs de « b » satisfaisantes.

### Exemple 5

Pour les essais n° 1, 4, 8 et 10 on a testé la stabilité du procédé en faisant fonctionner le procédé de manière continue sur plusieurs jours.
Des échantillons ont été prélevés à 24 heures d'intervalle pour analyse. Les valeurs « b » ainsi déterminées sont récapitulées au tableau 4 ci-dessous.

**Tableau 4**

| **Jours** | **Essai 1** | **Essai 4** | **Essai 8** | **Essai 10** |
|---|---|---|---|---|
| 1 | 1,43 | 1,52 | 0,97 | 0,95 |
| 2 | 2,10 | 1,13 | 0,96 | 0,81 |
| 3 | 1,75 | 1,90 | 0,82 | 0,85 |
| 4 | 1,60 | 2,04 | 0,97 | 0,78 |
| 5 | 2,56 | 1,38 | 0,82 | 0,74 |
| 6 | 2,13 | 1,54 | 0,78 | 0,79 |
| 7 | 1,50 | 1,52 | 0,85 | 0,74 |
| 8 | 2,46 | 1,68 | 0,82 | 0,71 |
| 9 | 2,05 | 1,41 | 0,89 | 0,87 |
| 10 | 1,99 | 1,78 | 0,92 | 0,81 |
| 11 | 1,81 | 1,65 | 0,78 | 0,79 |
| 12 | 1,49 | 1,98 | 0,75 | 0,91 |
| 13 | 1,79 | 2,70 | 0,82 | 0,94 |
| 14 | 1,84 | 2,14 | 0,89 | 0,91 |
| 15 | 1,96 | 1,85 | 0,91 | 0,85 |
| Valeur moyenne | 1,92 | 1,75 | 0,86 | 0,83 |
| Ecart type | 0,37 | 0,38 | 0,07 | 0,08 |

Ce tableau établit clairement la stabilité du procédé selon l'invention, les mesures étant bien plus reproductibles que dans le cas de l'art antérieur.

## Revendications

1. Procédé de fabrication de cristaux d'acide succinique à partir d'un milieu de fermentation contenant de l'acide succinique, comprenant les étapes de :
a) porter le milieu de fermentation à un pH compris entre 1,0 et 4,0,
b) cristalliser l'acide succinique du milieu de fermentation issu de l'étape a) de manière à former des cristaux d'acide succinique, séparer ensuite les cristaux d'acide succinique des eaux mères de cristallisation puis laver les cristaux obtenus avec de l'eau,
c) dissoudre les cristaux d'acide succinique obtenus suite à l'étape b) dans de l'eau à une température comprise entre 30 °C et 70 °C de manière à obtenir une solution contenant de l'acide succinique dissout,
d) purifier la solution d'acide succinique obtenue à l'étape c) par un traitement sur charbon actif et sur résine échangeuses d'ions,
e) cristalliser l'acide succinique contenu dans la solution obtenue à l'étape d) de manière à former des cristaux d'acide succinique, séparer ensuite les cristaux d'acide succinique des eaux mères de cristallisation puis laver les cristaux obtenus avec de l'eau,
f) sécher les cristaux d'acide succinique à une humidité inférieure à 0,5 % et les refroidir à une température inférieure à 30°C,
**caractérisé en ce qu'**on introduit avant et / ou pendant l'étape b) au moins un tensioactif, préférentiellement pendant l'étape b).

2. Procédé selon la revendication 1, **caractérisé en ce que** ledit tensioactif est choisi parmi les tensioactifs non ioniques, et préférentiellement parmi les polysorbates ayant un HLB supérieur à 15, ou parmi les tensioactifs à base de copolymère bloc d'oxyde d'alkylène.

3. Procédé selon une des revendications précédentes, **caractérisé en ce que** la première étape a) consiste donc à porter le milieu de fermentation à un pH compris entre 1,5 et 3,5 et préférentiellement entre 2,0 et 3,0.

4. Procédé selon une des revendications précédentes, **caractérisé en ce que** le milieu de fermentation contient des bactéries choisies parmi les souches bactérienne du genre *Mannheimia, Anaerobiospirillum, Bacillus,* ou *Escherichia,* ou parmi les souches fongiques.

5. Procédé selon une des revendications précédentes, **caractérisé en ce que** dans la deuxième étape b) la cristallisation intervient soit en batch soit en continu et par refroidissement.

6. Procédé selon une des revendications précédentes, **caractérisé en ce que** la séparation des cristaux des eaux mères est réalisée par filtration ou centrifugation.

7. Procédé selon une des revendications précédentes, **caractérisé en ce que** le lavage est réalisé avec de l'eau, préférentiellement avec de l'eau déminéralisée, à une température comprise entre 15 °C et 25 °C, préférentiellement à environ 20°C.

8. Procédé selon une des revendications précédentes, **caractérisé en ce que** une partie des eaux mères de cristallisation et de lavage est recyclée en tête de l'étape b).

9. Procédé selon une des revendications précédentes, **caractérisé en ce que** l'étape c) consiste à dissoudre les cristaux d'acide succinique dans de l'eau manière à obtenir une matière sèche comprise entre 5 % et 50 % en poids du poids total de ladite solution, préférentiellement entre 10 % et 20 %.

10. Procédé selon une des revendications précédentes, **caractérisé en ce qu'**au cours de l'étape d) le charbon actif est sous forme de poudre ou granulaire, plus préférentiellement sous forme granulaire.

11. Procédé selon une des revendications précédentes, **caractérisé en ce qu'**au cours de l'étape e) on recycle la totalité des eaux mères de cristallisation et de lavage en tête de l'étape b).

12. Procédé selon une des revendications précédentes, **caractérisé en ce que** l'étape f) consiste à sécher les cristaux d'acide succinique à une humidité inférieure à 0,5 %, préférentiellement inférieure à 0,4 %, très préférentiellement inférieure à 0,3 % en poids d'eau par rapport au poids sec d'acide succinique, et à les refroidir à une température inférieure à 30°C, préférentiellement inférieure à 25 °C.

13. Cristaux d'acide succinique obtenu à partir d'un milieu de fermentation contenant de l'acide succinique, **caractérisée en ce qu'**ils présentent un indice de coloration b, mesuré dans le référentiel L, a, b, inférieur ou égal à 1,00 préférentiellement inférieur ou égal à 0,90, très préférentiellement inférieur ou égal à 0,80 et **en ce qu'**ils présentent un indice de sphéricité, tel que mesuré selon le test de Rittenhouse, au moins égal à 0,70, préférentiellement au moins égal à 0,75, et très préférentiellement au moins égal à 0,85.

14. Cristaux d'acide succinique selon la revendication précédente, **caractérisés en ce qu'**ils présentent une teneur en sucres réducteurs inférieure à 20 ppm, préférentiellement inférieure à 10 ppm, par rapport au poids total de cristaux anhydres.

15. Composition contenant des cristaux d'acide succinique selon l'une des revendications 13 ou 14.

## Patentansprüche

1. Verfahren zur Herstellung von Bernsteinsäurekristallen basierend auf einem Bernsteinsäure enthaltenden Fermentationsmedium, welches die folgenden Schritte umfasst:
a) Einstellung des Fermentationsmediums auf einen pH-Wert zwischen 1,0 und 4,0,
b) Kristallisation der Bernsteinsäure des Fermentationsmediums aus Schritt a), so dass Bernsteinkristalle gebildet werden, anschließend Trennen der Bernsteinsäurekristalle von den Kristallisations-Mutterlaugen, dann Waschen der erhaltenen Kristalle mit Wasser,
c) Lösen der nach Schritt b) erhaltenen Bernsteinsäurekristalle in Wasser bei einer Temperatur von 30-70 °C zum Erhalt einer Lösung, welche gelöste Bernsteinsäure enthält,
d) Reinigung der in Schritt c) erhaltenen Bernsteinsäurelösung durch eine Behandlung auf Aktivkohle und auf Ionenaustauschharz,
e) Kristallisation der in der in Schritt d) erhaltenen Lösung enthaltenen Bernsteinsäurelösung zur Bildung von Bernsteinsäurekristallen, anschließend Trennen der Bernsteinsäurekristalle von den Kristallisations-Mutterlaugen, dann Waschen der erhaltenen Kristalle mit Wasser,
f) Trocknen der Bernsteinsäurekristalle bei einer Feuchtigkeit kleiner 0,5 % und Abkühlen auf eine Temperatur kleiner 30 °C,
**dadurch gekennzeichnet, dass** vor und/oder während Schritt b) wenigstens ein oberflächenaktives Mittel eingeführt wird, bevorzugt während Schritt b).

2. Verfahren nach Anspruch 1, **dadurch gekennzeichnet, dass** das oberflächenaktive Mittel gewählt ist aus nicht-ionischen oberflächenaktiven Mitteln, und bevorzugt aus Polysorbaten mit einem HLB größer 15 oder aus oberflächenaktiven Mitteln auf der Basis eines Alkylenoxid-Blockcopolymers.

3. Verfahren nach einem der vorhergehenden Ansprüche, **dadurch gekennzeichnet, dass** der erste Schritt a) darin besteht, das Fermentationsmedium auf einen pH-Wert von 1,5 bis 3,5 einzustellen, bevorzugt von 2,0 bis 3,0.

4. Verfahren nach einem der vorhergehenden Ansprüche, **dadurch gekennzeichnet, dass** das Fermentationsmedium Bakterien umfasst, die gewählt sind aus Bakterienstämmen der Gattungen *Mannheimia, Anaerobiospirillum, Bacillus* oder *Escherichia* oder aus Pilzstämmen.

5. Verfahren nach einem der vorhergehenden Ansprüche, **dadurch gekennzeichnet, dass** im zweiten Schritt b) die Kristallisation als Batch oder fortlaufend und durch Kühlung erfolgt.

6. Verfahren nach einem der vorhergehenden Ansprüche, **dadurch gekennzeichnet, dass** die Trennung der Kristalle von den Mutterlaugen durch Filtration oder Zentrifugieren erfolgt.

7. Verfahren nach einem der vorhergehenden Ansprüche, **dadurch gekennzeichnet, dass** das Waschen mit Wasser erfolgt, bevorzugt mit demineralisiertem Wasser, bei einer Temperatur von 15 bis 25 °C, bevorzugt von ungefähr 20 °C.

8. Verfahren nach einem der vorhergehenden Ansprüche, **dadurch gekennzeichnet, dass** ein Teil der Kristallisations- und Wasch-Mutterlaugen aus Schritt b) recycelt wird.

9. Verfahren nach einem der vorhergehenden Ansprüche, **dadurch gekennzeichnet, dass** Schritt c) darin besteht, die Bernsteinsäurekristalle in Wasser zu lösen, um eine Trockensubstanz mit 5 bis 50 Gewichtsprozent des Gesamtgewichts der Lösung zu erhalten, bevorzugt zwischen 10 und 20 %.

10. Verfahren nach einem der vorhergehenden Ansprüche, **dadurch gekennzeichnet, dass** im Verlauf von Schritt d) die Aktivkohle in Form von Pulver oder Granulat vorliegt, stärker bevorzugt als Granulat.

11. Verfahren nach einem der vorhergehenden Ansprüche, **dadurch gekennzeichnet, dass** im Verlauf von Schritt e) die Gesamtheit der Kristallisations- und Wasch-Mutterlaugen aus Schritt b) recycelt wird.

12. Verfahren nach einem der vorhergehenden Ansprüche, **dadurch gekennzeichnet, dass** Schritt f) darin besteht, die Bernsteinsäurekristalle bei einer Feuchtigkeit kleiner 0,5 %, bevorzugt kleiner 0,4 %, stark bevorzugt kleiner 0,3 Gewichtsprozent Wasser in Bezug auf das Trockengewicht der Bernsteinsäure zu trocknen und sie auf eine Temperatur kleiner 30 °C, bevorzugt kleiner 25 °C abzukühlen.

13. Bernsteinsäurekristalle, welche basierend auf einem Bernsteinsäure enthaltenden Fermentationsmedium erhalten wurden, **dadurch gekennzeichnet, dass** sie einen Färbungsindex b aufweisen, gemessen im Bezugsrahmen L, a, b, kleiner gleich 1,00, bevorzugt kleiner gleich 0,90, stark bevorzugt kleiner gleich 0,80 und dass sie einen nach Rittenhouse gemessenen Sphärizitätsindex aufweisen, der wenigstens gleich 0,70, bevorzugt wenigstens gleich 0,75 und stark bevorzugt wenigstens gleich 0,85 ist.

14. Bernsteinsäurekristalle nach dem vorhergehenden Anspruch, **dadurch gekennzeichnet, dass** sie einen Gehalt an reduzierenden Zuckern kleiner 20 ppm, bevorzugt kleiner 10 ppm, in Bezug auf das Gesamtgewicht an wasserfreien Kristallen aufweisen.

15. Zusammensetzung, welche Bernsteinsäurekristalle nach einem der Ansprüche 13 oder 14 umfasst.

## Claims

1. A process for producing succinic acid crystals from a fermentation medium containing succinic acid, comprising the steps of:
a) bringing the fermentation medium to a pH of between 1.0 and 4.0,
b) crystallizing the succinic acid from the fermentation medium resulting from step a) so as to form succinic acid crystals, then separating the succinic acid crystals from the crystallization mother liquors and then washing the obtained crystals with water,
c) dissolving the succinic acid crystals obtained following step b) in water at a temperature between 30 °C and 70 °C so as to obtain a solution containing dissolved succinic acid,
d) purifying the succinic acid solution obtained in step c) using a treatment on activated carbon and on ion exchange resin,
e) crystallizing the succinic acid contained in the solution obtained in step d) so as to form succinic acid crystals, subsequently separating the succinic acid crystals from the crystallization mother liquors and then washing the obtained crystals with water,
f) drying the succinic acid crystals to a moisture content of less than 0.5% and cooling them to a temperature below 30 °C,
**characterized in that** at least one surfactant is introduced before and/or during step b), preferentially during step b).

2. The process as claimed in claim 1, **characterized in that** said surfactant is chosen from non-ionic surfactants, and preferentially from polysorbates having an HLB greater than 15, or from surfactants based on alkylene oxide block copolymer.

3. The process as claimed in either of the preceding claims, **characterized in that** the first step a) thus consists in bringing the fermentation medium to a pH of between 1.5 and 3.5 and preferentially between 2.0 and 3.0.

4. The process as claimed in one of the preceding claims, **characterized in that** the fermentation medium contains bacteria chosen from bacterial strains of the *Mannheimia, Anaerobiospirillum, Bacillus* or *Escherichia* genus, or from fungal strains.

5. The process as claimed in one of the preceding claims, **characterized in that**, in the second step b), the crystallization occurs either batchwise or continuously and by cooling.

6. The process as claimed in one of the preceding claims, **characterized in that** the separation of the crystals from the mother liquors is carried out by filtration or centrifugation.

7. The process as claimed in one of the preceding claims, **characterized in that** the washing is carried out with water, preferentially with demineralized water, at a temperature of between 15°C and 25°C, preferentially at approximately 20°C.

8. The process as claimed in one of the preceding claims, **characterized in that** a part of the crystallization and washing mother liquors is recycled to the top of step b).

9. The process as claimed in one of the preceding claims, **characterized in that** step c) consists in dissolving the succinic acid crystals in water so as to obtain a dry matter content of between 5% and 50% by weight of the total weight of said solution, preferentially between 10% and 20%.

10. The process as claimed in one of the preceding claims, **characterized in that**, during step d), the activated carbon is in powder form or granular form, more preferentially in granular form.

11. The process as claimed in one of the preceding claims, **characterized in that**, during step e), all of the crystallization and washing mother liquors are recycled to the top of step b).

12. The process as claimed in one of the preceding claims, **characterized in that** step f) consists in drying the succinic acid crystals to a moisture content of less than 0.5%, preferentially less than 0.4%, very preferentially less than 0.3% by weight of water relative to the dry weight of succinic acid, and in cooling them to a temperature below 30°C, preferentially below 25°C.

13. Succinic acid crystals obtained from a fermentation medium containing succinic acid, **characterized in that** they have a color index b, measured in the reference system L, a, b, of less than or equal to 1.00, preferentially less than or equal to 0.90, very preferentially less than or equal to 0.80 and **in that** they have a sphericity index, as measured according to the Rittenhouse test, at least equal to 0.70, preferentially at least equal to 0.75, and very preferentially at least equal to 0.85.

14. The succinic acid crystals as claimed in the preceding claim, **characterized in that** they have a reducing sugar content of less than 20 ppm, preferentially less than 10 ppm, relative to the total weight of anhydrous crystals.

15. A composition containing succinic acid crystals as claimed in one of claims 13 or 14.
